# EUROPÄISCHE PATENTSCHRIFT

(11) **EP 3 721 837 B1**
(45) Veröffentlichungstag und Bekanntmachung des Hinweises auf die Patenterteilung: **23.10.2024**
(21) Anmeldenummer: 20164509.0
(22) Anmeldetag: 20.03.2020
(51) Int. Cl.: A61F 2/32, A61F 2/36

(54) **HÜFTGELENKENDOPROTHESENSYSTEM**
ACETABULAR ENDOPROSTHESIS SYSTEM
SYSTÈME D'ENDOPROTHÈSE D'ARTICULATION DE LA HANCHE

(30) Priorität: 21.03.2019 DE 102019107244
(43) Veröffentlichungstag der Anmeldung: 14.10.2020
(73) Patentinhaber: Aesculap AG, 78532 Tuttlingen (DE)
(72) Erfinder: REU, Gerhard, 78532 Tuttlingen (DE)
(74) Vertreter: Hoeger, Stellrecht & Partner Patentanwälte mbB

(56) Entgegenhaltungen:
- EP-B2- 0 137 040
- US-A1- 2006 217 815

## Beschreibung

Die vorliegende Erfindung betrifft ein Hüftgelenkendoprothesensystem umfassend eine Gelenkpfanne und einen zur Gelenkpfanne korrespondierend ausgebildeten Gelenkkopf zur Ausbildung eines Kugelgelenks, welcher Gelenkkopf mit einem Femurschaft koppelbar ist, wobei die Gelenkpfanne eine Gelenkpfannenschale mit einer Einsatzausnehmung aufweist, in die ein Gelenkpfanneneinsatz mit einer hohlkugeligen Gelenkpfannenfläche eingesetzt ist, wobei der Gelenkkopf zweiteilig ausgebildet ist und mindestens einen mit dem Femurschaft koppelbaren Kugelkopf und eine Gelenkkopfschale umfasst und wobei die Gelenkkopfschale einerseits mit dem mindestens einen Kugelkopf kugelgelenkig und andererseits mit dem Gelenkpfanneneinsatz kugelgelenkig gekoppelt ist.

Hüftgelenkendoprothesensysteme der eingangs beschriebenen Art kommen zunehmend bei Hüftrevisionseingriffen zum Einsatz. Die Besonderheit dieser Hüftgelenkendoprothesensysteme ist ihre doppelte Beweglichkeit durch die kugelgelenkige Verbindung des Gelenkkopfes mit der Gelenkkopfschale einerseits und des Gelenkpfanneneinsatzes mit der Gelenkkopfschale andererseits. Derartige Mobilitätskonzepte bieten gewisse Vorteile hinsichtlich der Gelenkstabilität, und zwar insbesondere bei defizitärer Weichteilsituation.

Konzeptbedingt kommt es bei derartigen Hüftgelenkendoprothesensystemen zudem jedoch zu einem praktisch unvermeidbaren Kontakt zwischen der Gelenkkopfschale und dem Femurschaft, insbesondere einem Prothesenhals desselben. Ein solcher Kontakt wird auch als "Impingement" bezeichnet.

Durch das Impingement kann es in unerwünschter Weise zu Beschädigungen an den miteinander in Kontakt tretenden Komponenten des Hüftgelenkendoprothesensystems kommen, also insbesondere zu Beschädigungen an der Gelenkkopfschale.

Aus der EP 0 137 040 B2 ist ein Kugelpfannenlager für ein künstliches Gelenk bekannt. In der US 2006/0217815 A1 ist ein modularer Prothesenkopf offenbart.

Es ist daher eine Aufgabe der vorliegenden Erfindung ein Hüftgelenkendoprothesensystem der eingangs beschriebenen Art so zu verbessern, dass die Gefahr von Beschädigungen, insbesondere der Gelenkkopfschale, minimiert wird.

Diese Aufgabe wird bei einem Hüftgelenkendoprothesensystem der eingangs beschriebenen Art erfindungsgemäß dadurch gelöst, dass der mindestens eine Kugelkopf ein Schutzelement umfasst zum Begrenzen einer Bewegung der Gelenkkopfschale in Richtung auf den Femurschaft.

Die vorgeschlagene Weiterbildung eines Hüftgelenkendoprothesensystems der eingangs beschriebenen Art ermöglicht es insbesondere, die Gefahr von Beschädigungen der Gelenkkopfschale zu minimieren. Dies wird insbesondere dadurch erreicht, dass das Schutzelement eine Bewegung der Gelenkkopfschale in Richtung auf den Femurschaft begrenzt. Insbesondere kann eine Oberfläche des Schutzelements derart ausgebildet sein beziehungsweise werden, dass eine Gefahr von Beschädigungen der Gelenkkopfschale bei einem Kontakt mit dem Schutzelement im Vergleich zu einem Kontakt der Gelenckopfschale mit dem Femurschaft oder einem Femurhals desselben, wenn kein Schutzelement vorhanden wäre, deutlich verringert ist. Das vorgeschlagene Hüftgelenkendoprothesensystem kann insbesondere mit bereits implantierten Femurschäften kombiniert werden, deren Prothesenhälse eine Rauheit und insbesondere auch Kanten aufweisen können, die die Gefahr von Beschädigungen der Gelenkkopfschale bergen. Durch das Schutzelement wird ein Kontakt zwischen der Gelenkkopfschale und dem Femurschaft beziehungsweise einem Prothesenhals desselben verhindert. Eine Kombination des Hüftgelenkendoprothesensystems mit bereits implantierten Prothesenschäften ist insbesondere besonders schonend für einen Patienten, und zwar insbesondere dann, wenn der implantierte Prothesenschaft noch eine ausreichende Stabilität aufweist und gut im Femur verankert ist. Das Schutzelement kann insbesondere derart ausgebildet und geformt werden, dass eine Gefahr von Beschädigungen der Gelenkkopfschale minimal ist. Durch das Schutzelement kann also insbesondere ein nachteiliger Kontakt der Gelenkkopfschale mit dem Femurschaft beziehungsweise einem Femurhals oder Prothesenhals desselben vermieden werden. Der wie beschrieben nachteilige Kontakt der Gelenkkopfschale mit dem Femurschaft wird dann durch einen geeigneten Kontakt mit dem Schutzelement ersetzt. Das Schutzelement kann insbesondere unabhängig davon, ob ein neuer Prothesenschaft implantiert wird oder ein bereits implantierter Prothesenschaft weiter genutzt wird, eine Gefahr einer Beschädigung der Gelenkkopfschale minimieren, unabhängig davon, wie der Prothesenschaft ausgebildet ist. Dies hat insbesondere den Vorteil, dass das Hüftgelenkendoprothesensystem mit unterschiedlichsten Prothesenschaftmodellen eingesetzt werden kann, die aufgrund der Verschiedenartigkeit ihrer Halsgeometrien, also der Form des Femurhalses, undefiniert und für ein Impingement nachteilig sind, da bei diesen in der Regel nur ein Punktkontakt zwischen der Gelenkkopfschale und dem Prothesenschaft ermöglicht wird.

Einfach und kompakt ausbilden lässt sich das Hüftgelenkendoprothesensystem insbesondere dann, wenn das Schutzelement und der mindestens eine Kugelkopf einstückig ausgebildet sind. Beispielsweise kann das Schutzelement in Form einer vom mindestens einen Kugelkopf abstehenden Schutzhülse ausgebildet sein.

Vorteilhaft ist es, wenn der mindestens eine Kugelkopf einen Innenkonus aufweist zum Ausbilden einer, insbesondere selbsthemmenden, Konusverbindung mit einem korrespondierenden Femurkonus des Femurschafts. Der Kugelkopf kann auf diese Weise insbesondere kompatibel zu bestehenden Hüftgelenkendoprothesen ausgebildet werden, insbesondere kompatibel zu Femurschäften, die am Markt verfügbar und beispielsweise bereits implantiert sind.

Ein besonders guter Schutz der Gelenkkopfschale kann insbesondere dadurch erreicht werden, wenn das Schutzelement den Femurschaft hülsenförmig umgibt. So kann von allen Seiten, und zwar unabhängig von einer Bewegung der Gelenkkopfschale und des mindestens einen Gelenkkopfes relativ zueinander, ein Kontakt zwischen der Gelenkkopfschale und dem Femurschaft, insbesondere einem Femurhals desselben, sicher verhindert werden kann. Es spielt dann keine Rolle mehr, wie der Femurschaft beziehungsweise der Femurhals ausgebildet ist. Eine Beschädigung der Gelenkkopfschale kann insbesondere vermieden werden durch eine entsprechende geeignete Ausgestaltung des Schutzelements.

Günstig ist es, wenn das Schutzelement vom Femurschaft, insbesondere einem Femurhals desselben, beabstandet angeordnet ist zur Ausbildung eines Ringraums zwischen dem Schutzelement und dem Femurschaft. Auf diese Weise kann insbesondere das Koppeln des Gelenkkopfes mit dem Femurschaft auf einfache Weise realisiert werden. Insbesondere kann so verhindert werden, dass das Schutzelement mit dem Femurschaft eine kraftschlüssige Kopplung eingeht, die eine Kopplung des Femurschafts mit dem mindestens einen Kugelkopf behindern oder beeinträchtigen würde.

Vorteilhaft ist es, wenn der Innenkonus eine Innenkonusfläche definiert und wenn eine in Richtung auf den Femurschaft weisende Schutzelementinnenfläche des Schutzelements von einer die Innenkonusfläche virtuell verlängernden Verlängerungsfläche beabstandet ist. So kann insbesondere sichergestellt werden, dass das Schutzelement nicht direkt in Kontakt mit dem Femurschaft oder einem Femurhals desselben kommen kann. Insbesondere wird so auch das Koppeln des mindestens einen Gelenkkopfes mit dem Femurschaft vereinfacht.

Günstigerweise beträgt ein Abstand des Schutzelements vom Femurschaft und/oder ein Abstand der virtuellen Verlängerungsfläche von der Innenkonusfläche maximal 1 mm. Eine solche dünnwandige Ausführung des Schutzelements ermöglicht es insbesondere, eine möglichst große Beweglichkeit der relativ zueinander beweglichen Teile zu erhalten. Mit anderen Worten wird so eine übermäßige Einschränkung eines Bewegungsumfangs des Hüftgelenkendoprothesensystems vermieden.

Vorzugsweise erweitert sich der Innenkonus im Übergangsbereich zum Schutzelement im Innendurchmesser. Insbesondere erweitert sich der Innenkonus im Übergangsbereich zum Schutzelement im Innendurchmesser einstufig. So lässt sich auf einfache Weise eine Beabstandung des Schutzelements vom Femurschaft, insbesondere von dessen Femurhals, sicherstellen. Beispielsweise kann so auch ein Ringraum zwischen dem Schutzelement und dem Femurschaft auf einfache Weise ausgebildet werden. Ferner kann so auch wirkungsvoll vermieden werden, dass das Schutzelement mit einem zum Innenkonus korrespondierenden Femurkonus des Femurschafts zusammenwirkt.

Vorteilhaft ist es, wenn ein freies, vom mindestens einen Kugelkopf weg weisendes Ende des Schutzelements wulstförmig verdickt ausgebildet ist. Insbesondere kann eine wulstförmige Verdickung von der Längsachse des Innenkonus weg weisen. Diese Ausgestaltung ermöglicht eine einfache und sichere Kopplung des mindestens einen Kugelkopfes mit einem bereits implantierten Femurschaft. Insbesondere kann so die Gefahr einer Schädigung von Weichteilgewebe bei der Kopplung minimiert werden.

Günstigerweise ist eine vom Femurschaft weg weisende Außenfläche des Schutzelements poliert. Eine derart bearbeitete Außenfläche des Schutzelements minimiert eine Gefahr einer Beschädigung der Gelenkkopfschale, und zwar insbesondere dann, wenn diese aus einem Kunststoff ausgebildet ist. Das Schutzelement kann in diesem Fall insbesondere aus einem metallischen Werkstoff ausgebildet sein. Das Polieren der Außenfläche dient insbesondere dazu, das Schutzelement mit einer minimalen Rauheit und insbesondere ohne jegliche Kanten auszubilden, die mit der Gelenkkopfschale bei einem Impingement in Kontakt treten und diese beschädigen könnten.

Um einen Bewegungsumfang des Hüftgelenkendoprothesensystems nicht unnötig einzuschränken, ist es vorteilhaft, wenn das Schutzelement eine Dicke aufweist, die maximal 1 mm beträgt.

Günstigerweise ist der mindestens eine Kugelkopf aus einem metallischen Werkstoff ausgebildet. Dies ermöglicht insbesondere eine sichere Verbindung mit einem Prothesenschaft, wenn dieser ebenfalls aus einem metallischen Werkstoff ausgebildet ist.

Vorteilhaft ist es, wenn die Gelenkkopfschale aus einem Kunststoff ausgebildet ist. So kann insbesondere eine optimale Gleitpaarung zwischen der Gelenckopfschale und dem mindestens einen Kugelkopf realisiert werden. Vorteilhafterweise ist der Kunststoff Polyethylen. Insbesondere kann der Kunststoff Polyethylen mit ultrahohem Molekulargewicht (UHMWPE) sein.

Gemäß einer weiteren bevorzugten Ausführungsform der Erfindung kann vorgesehen sein, dass eine Außenkontur der Gelenkkopfschale derart geformt ist, dass sie, wenn sie mit dem Schutzelement in Kontakt steht, das Schutzelement mindestens linienförmig, insbesondere flächig, berührt. Auf diese Weise kann die Gefahr einer Beschädigung der Gelenkkopfschale bei einem Impingement, also einem in Kontakt Treten mit dem Schutzelement, signifikant verringert werden.

Vorteilhaft ist es, wenn die Außenkontur der Gelenkkopfschale eine konische, in Richtung auf den Femurhals weisende Gelenkkopfschalenanschlagfläche aufweist und wenn eine erste Schnittlinie der Gelenkkopfschalenanschlagfläche mit einer Schnittebene, in der ein Gelenkzentrum der kugelgelenkigen Verbindung zwischen der Gelenkkopfschale und dem mindestens einen Kugelkopf liegt, parallel oder im Wesentlichen parallel zu einer zweiten Schnittlinie des Schutzelements mit der Schnittebene verläuft. Die beschriebene Ausgestaltung ermöglicht insbesondere einen linienförmigen, insbesondere flächigen, Kontakt zwischen der Gelenkkopfschale und dem Schutzelement bei einem Impingement.

Günstig ist es, wenn das Hüftgelenkendoprothesensystem zwei oder mehr Kugelköpfe umfasst und wenn Innenkonen der zwei oder mehr Kugelköpfe unterschiedlich tief ausgebildet sind. Diese Weiterbildung ermöglicht es insbesondere, bei einer Revision einer Hüftgelenkendoprothese den Femurschaft im Femur des Patienten zu belassen und durch geeignete Auswahl eines der zwei oder mehr Kugelköpfe eine Position eines Gelenkzentrums des Hüftgelenkendoprothesensystems in gewünschter Weise relativ zum Femurschaft zu positionieren. Insbesondere können die Innenkonen derart unterschiedlich tief ausgebildet sein, dass eine Position des Gelenkzentrums in gewünschter Weise vorgegeben werden kann.

Vorteilhaft ist es, wenn ein von den zwei oder mehr Kugelköpfen definierter Außendurchmesser identisch oder im Wesentlichen identisch ist. Dieser ermöglicht es insbesondere, eine hohe Variabilität des Hüftgelenkendoprothesensystems zu erreichen. Eine Position des Gelenkzentrums der Hüftgelenkendoprothese lässt sich dann auf einfache Weise lediglich durch entsprechende Auswahl eines Kugelkopfes variieren. Alle anderen Komponenten des Hüftgelenkendoprothesensystems können dabei unverändert bleiben.

Ferner ist es vorteilhaft, wenn die kugelgelenkige Verbindung zwischen der Gelenkkopfschale und dem mindestens einen Kugelkopf ein erstes Rotationszentrum definiert, wenn die kugelgelenkige Verbindung zwischen der Gelenckopfschale und dem Gelenkpfanneneinsatz ein zweites Rotationszentrum definiert und wenn das erste Rotationszentrum und das zweite Rotationszentrum identisch oder im Wesentlichen identisch sind. Mit anderen Worten kann auf die beschriebene Weise ein einziges Rotationszentrum definiert werden, um das die miteinander kugelgelenkig gekoppelten Komponenten des Hüftgelenkendoprothesensystems relativ zueinander rotierbar sind.

Gemäß einer weiteren bevorzugten Ausführungsform der Erfindung kann vorgesehen sein, dass das Hüftgelenkendoprothesensystem einen Femurschaft mit einem Femurhals umfasst, an welchem Femurhals ein Femurkonus ausgebildet ist, welcher Femurkonus ein freies Ende des Femurhalses bildet. Ein solcher Femurschaft kann insbesondere über eine Konuskopplung mit dem mindestens einen Kugelkopf des Hüftgelenkendoprothesensystems axial und drehfest gekoppelt werden, beispielsweise über eine selbsthemmende Konusverbindung.

Vorteilhaft ist es, wenn ein Verhältnis einer Schutzelementhaltekraft zwischen dem Schutzelement und dem Femurkonus und/oder dem Femurhals und einer Konushaltekraft der Konusverbindung andererseits in einem Bereich von etwa 1:20 bis etwa 1:500 liegt. Insbesondere kann das Verhältnis in einem Bereich von etwa 1: 50 bis etwa 1:200 liegen. Insbesondere kann es etwa 1: 100 betragen. Auf diese Weise kann insbesondere sichergestellt werden, dass eine Kopplung des mindestens einen Kugelkopfes mit dem Femurschaft nicht durch eine Verbindung zwischen dem Schutzelement und dem Femurschaft realisiert wird, sondern dass die Konusverbindung im Wesentlichen durch die definierte Konushaltekraft bewirkt wird.

Ferner kann es günstig sein, wenn ein Verformungsverhältnis einer Verformungskraft zum Verformen des Schutzelements durch Anschlagen der Gelenckopfschale einerseits und einer Haltekraft der Konusverbindung andererseits in einem Bereich von etwa 1:20 bis etwa 1:500 liegt. Insbesondere kann das Verformungsverhältnis in einem Bereich von etwa 1:50 bis etwa 1:200 liegen. Insbesondere kann es etwa 1:100 betragen. Ein Verformungsverhältnis in den angegebenen Bereichen vorzusehen hat insbesondere den Vorteil, dass eine Verformung des Schutzelements leichter möglich ist als den mindestens einen Kugelkopf vom Femurschaft zu lösen. So kann eine sichere und dauerhafte Verbindung zwischen dem mindestens einen Kugelkopf und dem Femurschaft erreicht werden.

Die vorstehende Beschreibung umfasst somit insbesondere die nachfolgend in Form durchnummerierter Sätze definierten Ausführungsformen von Hüftgelenkendoprothesensystemen :
1. Hüftgelenkendoprothesensystem (10) umfassend eine Gelenkpfanne (12) und einen zur Gelenkpfanne (12) korrespondierend ausgebildeten Gelenkkopf (14) zur Ausbildung eines Kugelgelenks (16), welcher Gelenckopf (14) mit einem Femurschaft (18) koppelbar ist, wobei die Gelenkpfanne (12) eine Gelenkpfannenschale (20) mit einer Einsatzausnehmung (22) aufweist, in die ein Gelenkpfanneneinsatz (24) mit einer hohlkugeligen Gelenkpfannenfläche (30) eingesetzt ist, wobei der Gelenkkopf (14) zweiteilig ausgebildet ist und mindestens einen mit dem Femurschaft (18) koppelbaren Kugelkopf (32, 32a, 32b, 32c, 32d) und eine Gelenkkopfschale (34) umfasst und wobei die Gelenkkopfschale (34) einerseits mit dem mindestens einen Kugelkopf (32, 32a, 32b, 32c, 32d) kugelgelenkig und andererseits mit dem Gelenkpfanneneinsatz (24) kugelgelenkig gekoppelt ist, dadurch gekennzeichnet, dass der mindestens eine Kugelkopf (32, 32a, 32b, 32c, 32d) ein Schutzelement (54) umfasst zum Begrenzen einer Bewegung der Gelenkkopfschale (34) in Richtung auf den Femurschaft (18).
2. Hüftgelenkendoprothesensystem nach Satz 1, dadurch gekennzeichnet, dass das Schutzelement (54) und der mindestens eine Kugelkopf (32, 32a, 32b, 32c, 32d) einstückig ausgebildet sind.
3. Hüftgelenkendoprothesensystem nach einem der voranstehenden Sätze, dadurch gekennzeichnet, dass der mindestens eine Kugelkopf (32, 32a, 32b, 32c, 32d) einen Innenkonus (44, 44a, 44b, 44c, 44d) aufweist zum Ausbilden einer, insbesondere selbsthemmenden, Konusverbindung mit einem korrespondierenden Femurkonus (50) des Femurschafts (18).
4. Hüftgelenkendoprothesensystem nach Satz 3, dadurch gekennzeichnet, dass das Schutzelement (54) den Femurschaft (18) hülsenförmig umgibt.
5. Hüftgelenkendoprothesensystem nach Satz 3 oder 4, dadurch gekennzeichnet, dass das Schutzelement (54) vom Femurschaft (18), insbesondere einem Femurhals (48) desselben, beabstandet angeordnet ist zur Ausbildung eines Ringraums (56) zwischen dem Schutzelement (54) und dem Femurschaft (18).
6. Hüftgelenkendoprothesensystem nach einem der Sätze 3 bis 5, dadurch gekennzeichnet, dass der Innenkonus (44, 44a, 44b, 44c, 44d) eine Innenkonusfläche (58) definiert und dass eine in Richtung auf den Femurschaft weisende Schutzelementinnenfläche (60) des Schutzelements (54) von einer die Innenkonusfläche (58) virtuell verlängernden Verlängerungsfläche beabstandet ist.
7. Hüftgelenkendoprothesensystem nach Satz 5 oder 6, dadurch gekennzeichnet, dass ein Abstand (64) des Schutzelements (54) vom Femurschaft (18) und/oder ein Abstand (66) der virtuellen Verlängerungsfläche (62) von der Innenkonusfläche (58) maximal 1 mm beträgt.
8. Hüftgelenkendoprothesensystem nach einem der Sätze 3 bis 7, dadurch gekennzeichnet, dass sich der Innenkonus (44, 44a, 44b, 44c, 44d) im Übergangsbereich zum Schutzelement (54) im Innendurchmesser erweitert, insbesondere einstufig.
9. Hüftgelenkendoprothesensystem nach einem der voranstehenden Sätze, dadurch gekennzeichnet, dass ein freies, vom mindestens einen Kugelkopf (32, 32a, 32b, 32c, 32d) weg weisendes Ende (70) des Schutzelements (54) wulstförmig verdickt ausgebildet ist, insbesondere von einer Längsachse (46) des Innenkonus (44) weg weisend.
10. Hüftgelenkendoprothesensystem nach einem der voranstehenden Sätze, dadurch gekennzeichnet, dass eine vom Femurschaft (18) weg weisende Außenfläche (74) des Schutzelements (54) poliert ist.
11. Hüftgelenkendoprothesensystem einem der voranstehenden Sätze, dadurch gekennzeichnet, dass das Schutzelement (54) eine Dicke (72) aufweist, die maximal 1 mm beträgt.
12. Hüftgelenkendoprothesensystem nach einem der voranstehenden Sätze, dadurch gekennzeichnet, dass der mindestens eine Kugelkopf (32, 32a, 32b, 32c, 32d) aus einem metallischen Werkstoff ausgebildet ist.
13. Hüftgelenkendoprothesensystem nach einem der voranstehenden Sätze, dadurch gekennzeichnet, dass die Gelenkkopfschale (34) aus einem Kunststoff ausgebildet ist, insbesondere aus Polyethylen, weiter insbesondere aus Polyethylen mit ultrahohem Molekulargewicht (UHMWPE).
14. Hüftgelenkendoprothesensystem nach einem der voranstehenden Sätze, dadurch gekennzeichnet, dass eine Außenkontur der Gelenkkopfschale (34) derart geformt ist, dass sie, wenn sie mit dem Schutzelement (54) in Kontakt steht, das Schutzelement (54) mindestens linienförmig, insbesondere flächig, berührt.
15. Vorrichtung nach Satz 14, dadurch gekennzeichnet, dass die Außenkontur der Gelenkkopfschale (34) eine konische, in Richtung auf den Femurhals (48) weisende Gelenkkopfschalenanschlagfläche (52) aufweist und dass eine erste Schnittlinie (76) der Gelenkkopfschalenanschlagfläche (52) mit einer Schnittebene, in der ein Gelenkzentrum (40) der kugelgelenkigen Verbindung zwischen der Gelenkkopfschale (34) und dem mindestens einen Kugelkopf (32, 32a, 32b, 32c, 32d) liegt, parallel oder im Wesentlichen parallel zu einer zweiten Schnittlinie (78) des Schutzelements (54) mit der Schnittebene verläuft.
16. Hüftgelenkendoprothesensystem nach einem der Sätze 3 bis 15, dadurch gekennzeichnet, dass das Hüftgelenkendoprothesensystem (10) zwei oder mehr Kugelköpfe (12, 12a, 12b, 12c 12d) umfasst und dass Innenkonen (44, 44a, 44b, 44c, 44d) der zwei oder mehr Kugelköpfe (32, 32a, 32b, 32c, 32d) unterschiedlich tief ausgebildet sind.
17. Hüftgelenkendoprothesensystem nach Satz 16, dadurch gekennzeichnet, dass ein von den zwei oder mehr Kugelköpfen (32, 32a, 32b, 32c, 32d) definierter Außendurchmesser (84) identisch oder im Wesentlichen iden-tisch ist.
18. Hüftgelenkendoprothesensystem nach einem der voranstehenden Sätze, dadurch gekennzeichnet, dass die kugelgelenkige Verbindung zwischen der Gelenkkopfschale (34) und dem mindestens einen Kugelkopf (32, 32a, 32b, 32c, 32d) ein erstes Rotationszentrum (40) definiert, dass die kugelgelenkige Verbindung zwischen der Gelenkkopfschale (34) und dem Gelenkpfanneneinsatz (24) ein zweites Rotationszentrum (40) definiert und dass das erste Rotationszentrum (40) und das zweite Rotationszentrum (40) identisch oder im Wesentlichen identisch sind.
19. Hüftgelenkendoprothesensystem nach einem der voranstehenden Sätze, gekennzeichnet durch einen Femurschaft (18) mit einem Femurhals (48), an welchem ein Femurkonus (50) ausgebildet ist, welcher Femurkonus (50) ein freies Ende des Femurhalses (48) bildet.
20. Hüftgelenkendoprothesensystem nach Satz 19, dadurch gekennzeichnet, dass ein Verhältnis einer Schutzelementhaltekraft zwischen dem Schutzelement (54) und dem Femurkonus (50) und/oder dem Femurhals (48) einerseits und einer Konushaltekraft der Konusverbindung andererseits in einem Bereich von etwa 1:20 bis etwa 1:500 liegt, insbesondere in einem Bereich von etwa 1:50 bis etwa 1:200, weiter insbesondere etwa 1:100 beträgt.
21. Hüftgelenkendoprothesensystem nach Satz 19 oder 20, dadurch gekennzeichnet, dass ein Verformungsverhältnis einer Verformungskraft zum Verformen des Schutzelements (54) durch Anschlagen der Gelenkkopfschale (34) einerseits und einer Haltekraft der Konusverbindung andererseits in einem Bereich von etwa 1:20 bis etwa 1:500 liegt, insbesondere in einem Bereich von etwa 1:50 bis etwa 1:200, weiter insbesondere etwa 1:100 beträgt.

Die nachfolgende Beschreibung bevorzugter Ausführungsformen der Erfindung dient im Zusammenhang mit der Zeichnung der näheren Erläuterung. Es zeigen:
- Figur 1:: eine schematische perspektivische Gesamtansicht eines Ausfüh-rungsbeispiels eines Hüftgelenkendoprothesensystems;
- Figur 2:: eine schematische perspektivische Explosionsdarstellung des Hüftgelenkendoprothesensystems aus Figur 1;
- Figur 3:: eine Schnittansicht längs Linie 3-3 in Figur 2;
- Figur 4:: eine schematische Schnittansicht ähnlich Figur 3 eines weiteren Ausführungsbeispiels eines Kugelkopfs mit an diesem gekoppel-tem Femurschaft;
- Figur 5:: eine schematische Schnittansicht analog Figur 4 eines weiteren Ausführungsbeispiels eines Kugelkopfs mit an diesem gekoppel-tem Femurschaft;
- Figur 6:: eine schematische Schnittansicht analog Figur 4 eines weiteren Ausführungsbeispiels eines Kugelkopfs mit an diesem gekoppel-tem Femurschaft; und
- Figur 7:: eine schematische Schnittansicht analog Figur 4 eines weiteren Ausführungsbeispiels eines Kugelkopfs mit an diesem gekoppel-tem Femurschaft.

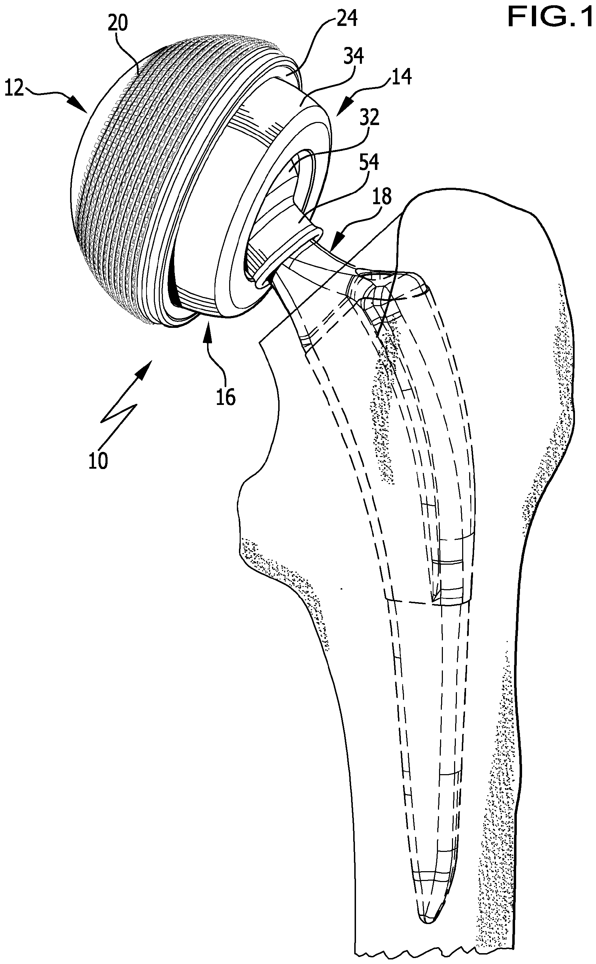

Ein erstes Ausführungsbeispiel eines Hüftgelenkendoprothesensystems 10 ist schematisch in Figur 1 dargestellt. Es umfasst eine Gelenkpfanne 10 und einen zu dieser korrespondierend ausgebildeten Gelenkkopf 14 zur Ausbildung eines Kugelgelenks 16.

Der Gelenkkopf 14 ist mit einem Femurschaft 18 koppelbar, also lösbar verbindbar.

Bei einem Ausführungsbeispiel umfasst das Hüftgelenkendoprothesensystem 10 auch den Femurschaft 18.

Die Gelenkpfanne 12 umfasst eine Gelenkpfannenschale 20, die in einem Becken eines Patienten in bekannter Weise verankerbar ist.

Die Gelenkpfannenschale 20 umfasst eine Einsatzausnehmung 22, in die ein Gelenkpfanneneinsatz 24 eingesetzt ist.

Bei einem Ausführungsbeispiel sind die Gelenkpfannenschale 20 und der Gelenkpfanneneinsatz 24 über eine selbsthemmende Konusverbindung miteinander gekoppelt. Zur Ausbildung dieser Konusverbindung weisen die Gelenkpfannenschale 20 eine innere Konusfläche 26 und der Gelenkpfanneneinsatz 24 eine zur Konusfläche 26 korrespondierende äußere Konusfläche 28 auf.

Die Gelenkpfannenschale 20 und der Gelenkpfanneneinsatz 24 sind bei einem Ausführungsbeispiel aus einem metallischen Werkstoff ausgebildet. Beispielsweise können beide Komponenten aus Titan ausgebildet sein.

Der Gelenkpfanneneinsatz 24, der in der Einsatzausnehmung 22 der Gelenkpfannenschale 20 aufgenommen ist, weist eine hohlkugelige Gelenkpfannenfläche 30 auf.

Der Gelenkkopf 14 ist zweiteilig ausgebildet. Er umfasst einen mit dem Femurschaft 18 koppelbaren Kugelkopf 32 und eine Gelenkkopfschale 34.

Die Gelenkkopfschale 34 weist eine kugelförmige Außenfläche 36 und eine hohlkugelige Innenfläche 38 auf.

Die Außenfläche 36 und die Innenfläche 38 sind konzentrisch zueinander ausgebildet und definieren ein gemeinsames Gelenkzentrum 40. Das Gelenkzentrum 40 bildet einen Mittelpunkt sowohl der Innenfläche 38 als auch der Außenfläche 36.

Die Gelenkkopfschale 34, deren Außenfläche 36 korrespondierend zur Gelenkpfannenfläche 30 ausgebildet ist, ist mit dem Gelenkpfanneneinsatz 24 kugelgelenkig gekoppelt.

Ferner ist die Gelenkkopfschale 34, deren Innenfläche 38 korrespondierend zu einer kugelförmigen Kugelkopffläche 42 des Kugelkopfes 32 ausgebildet ist, kugelgelenkig mit dem Kugelkopf 32 gekoppelt.

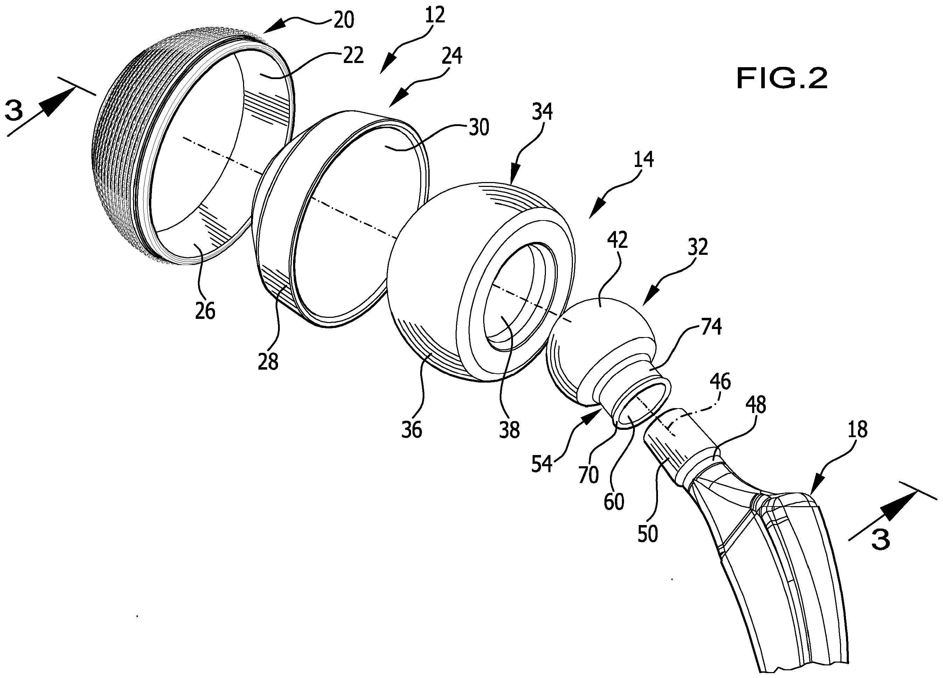

Bei dem in den Figuren 1 bis 3 schematisch dargestellten Ausführungsbeispiel des Hüftgelenkendoprothesensystems 10 bilden die Gelenkkopfschale 34 und der Gelenkpfanneneinsatz 24 eine erste Gleitpaarung. Ferner bilden die Gelenkkopfschale 34 und der Gelenkkopf 32 eine zweite Gleitpaarung.

Ferner ist bei dem in den Figuren 1 bis 3 dargestellten Ausführungsbeispiel des Hüftgelenkendoprothesensystems 10 die Gelenkkopfschale 34 aus einem Kunststoff ausgebildet. Bei einem Ausführungsbeispiel ist der Kunststoff Polyethylen. Bei einem weiteren Ausführungsbeispiel ist die Gelenkkopfschale 34 aus einem Polyethylen mit ultrahohem Molekulargewicht ausgebildet.

Bei einem Ausführungsbeispiel des Hüftgelenkendoprothesensystems 10 ist der Gelenkkopf 32 aus einem metallischen Werkstoff ausgebildet. Insbesondere ist bei einem Ausführungsbeispiel der Gelenkkopf 32 aus einem Implantatstahl ausgebildet.

Zum Koppeln des Gelenkkopfes 14 mit dem Femurschaft 18 dient eine Konusverbindung. Diese umfasst einen eine Längsachse 46 definierenden Innenkonus 44 am Kugelkopf 32.

Bei dem in den Figuren 1 bis 3 dargestellten Ausführungsbeispiel liegt das Gelenkzentrum 40 auf der Längsachse 46.

Der Femurschaft 18 umfasst einen Femurhals 48, an welchem ein Femurkonus 50 ausgebildet ist. Der Femurkonus 50 bildet ein freies Ende des Femurhalses 48.

Der Femurkonus 50 ist korrespondierend zum Innenkonus 44 ausgebildet und weist einen Konuswinkel auf, der im Zusammenwirken mit dem Innenkonus 44 die Ausbildung einer selbsthemmenden Konusverbindung zwischen dem Kugelkopf 32 und dem Femurschaft 18 ermöglicht. Der Kugelkopf 32 kann so auf einfache Weise drehfest und axial am Femurschaft 18 gesichert werden.

Die Gelenkkopfschale 34 weist eine in Richtung auf die Längsachse 46 hin weisende Gelenkkopfschalenanschlagfläche 52 auf. Diese ist konisch ausgebildet und erweitert sich von der Gelenkkopfschale 34 weg in Richtung auf den Femurschaft 18 hin.

Um einen direkten Kontakt zwischen der Gelenkkopfschale 34, insbesondere zwischen der Gelenkkopfschalenanschlagfläche 52, und dem Femurschaft 18, insbesondere dessen Femurhals 48, zu vermeiden, umfasst der Kugelkopf 32 ein Schutzelement 54. Es begrenzt eine Bewegung der Gelenkkopfschale 34 in Richtung auf den Femurschaft 18 beziehungsweise in Richtung auf die Längsachse 46 hin.

Bei dem in den Figuren 1 bis 3 dargestellten Ausführungsbeispiel sind das Schutzelement 54 und der Gelenkkopf einstückig ausgebildet.

Das Schutzelement 54 ist den Femurschaft 18 im Bereich des Femurhalses 48 hülsenförmig umgebend ausgebildet.

Das Schutzelement 54, das auch als Kragen des Kugelkopfes 32 oder als Schutzhülse bezeichnet werden kann, ist vom Femurschaft 18, insbesondere von dessen Femurhals 48, beabstandet angeordnet. Auf diese Weise wird ein Ringraum 56 zwischen dem Schutzelement 54 und dem Femurschaft 18 ausgebildet. Dieser Ringraum 56 ist bei dem in den Figuren 1 bis 3 dargestellten Ausführungsbeispiel in einer Richtung vom Kugelkopf 32 weg weisend geöffnet.

Der Innenkonus 44 definiert eine Innenkonusfläche 58. Ferner definiert das Schutzelement 54 eine in Richtung auf die Längsachse 46 hin weisende Schutzelementinnenfläche 60. Bei dem in den Figuren dargestellten Ausführungsbeispiel ist die Schutzelementinnenfläche 60 von einer die Innenkonusfläche 58 virtuell verlängernden konischen Verlängerungsfläche 62 beabstandet.

Ein Abstand 64 des Schutzelements 54 vom Femurschaft 18 beträgt maximal 1 mm. Ferner beträgt ein Abstand 66 der Verlängerungsfläche 62 von der Innenkonusfläche 58 maximal 1 mm.

Die Schutzelementinnenfläche 60 ist gegenüber der Innenkonusfläche 48 bezogen auf die Längsachse 46 etwas zurückgesetzt. Um dies zu erreichen, erweitert sich der Innenkonus 44 in einem Übergangsbereich 68 zwischen dem Innenkonus 44 und dem Schutzelement 54 im Innendurchmesser etwas, insbesondere einstufig.

Bei einem Ausführungsbeispiel ist ein freies, vom Kugelkopf 32 weg weisendes Ende 70 des Schutzelements 54 wulstförmig verdickt ausgebildet. Die wulstförmige Verdickung weist insbesondere von der Längsachse 46 des Innenkonus 44 weg.

Das Schutzelement 54 weist eine Hülsenwand mit einer Dicke 72 auf. Die Dicke 72 beträgt bei dem Ausführungsbeispiel maximal 1 mm.

Die Dicke 72 des Schutzelements 54 im Bereich des Endes 70 kann insbesondere mehr als 1 mm betragen. Dies ist insbesondere möglich und nicht nachteilig, da das wulstförmig verdickte Ende 70 einen Bewegungsbereich der Gelenkkopfschale 34 und des Kugelkopfes 32 relativ zueinander nicht beschränkt.

Eine von der Längsachse 46 weg weisende Außenfläche 74 des Schutzelements 54 ist poliert. Insbesondere weist die Außenfläche 74 eine minimale, allenfalls mikroskopische Rauheit auf sowie weder Ecken noch Kanten.

Ferner ist eine Außenkontur der Gelenkkopfschale 34 derart geformt, dass die Gelenkkopfschale 34, wenn sie mit dem Schutzelement 54 in Kontakt steht, das Schutzelement 54 zumindest linienförmig, insbesondere flächig berührt. Dies wird bei dem in den Figuren 1 bis 3 dargestellten Ausführungsbeispiel dadurch erreicht, dass die Außenkontur der Gelenkkopfschale 34 die konische Gelenkkopfschalenanschlagfläche 52 umfasst.

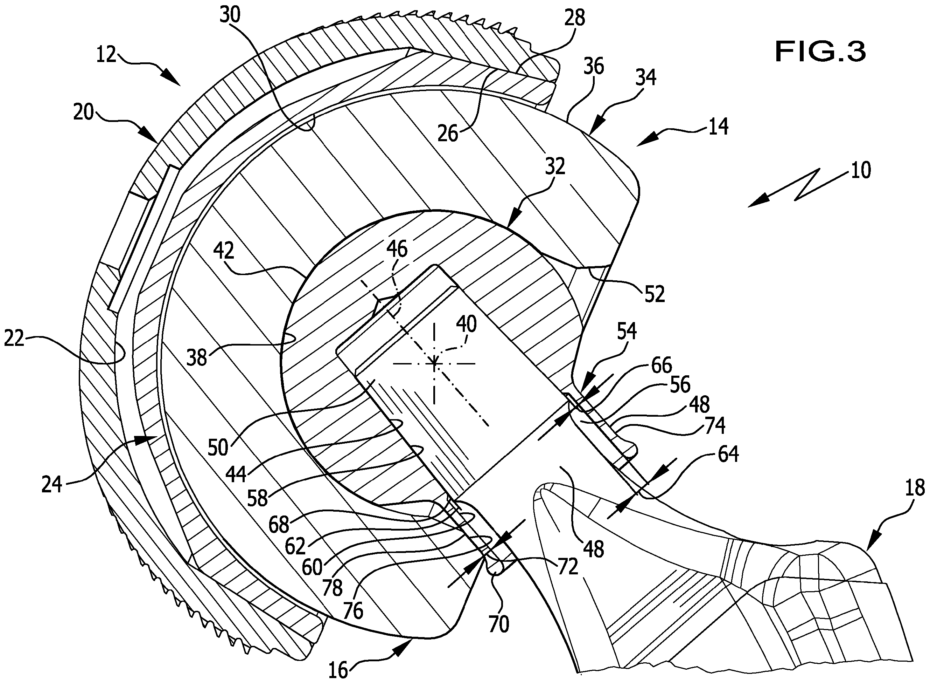

Ein Konuswinkel der Gelenkkopfschalenanschlagfläche 52 ist so gewählt, dass eine Schnittlinie derselben mit einer das Gelenkzentrum 40 enthaltenden Schnittebene eine erste Schnittlinie 76 definiert. Eine zweite Schnittlinie 78, die definiert wird durch die die Außenfläche 74 schneidende Schnittebene, verläuft parallel zur ersten Schnittlinie 76, wenn die Gelenkkopfschalenanschlagfläche 52 an der Außenfläche 74 anliegt. Dies ist schematisch in Figur 3 dargestellt.

Die kugelgelenkige Verbindung zwischen der Gelenkkopfschale 34 und dem Kugelkopf 32 definiert ein erstes Rotationszentrum. Ferner definiert die kugelgelenkige Verbindung zwischen der Gelenkkopfschale 34 und dem Gelenkpfanneneinsatz 24 ein zweites Rotationszentrum. Das erste und das zweite Rotationszentrum sind bei dem in den Figuren 1 bis 3 dargestellten Ausführungsbeispiel identisch und fallen mit dem Gelenkzentrum 40 zusammen.

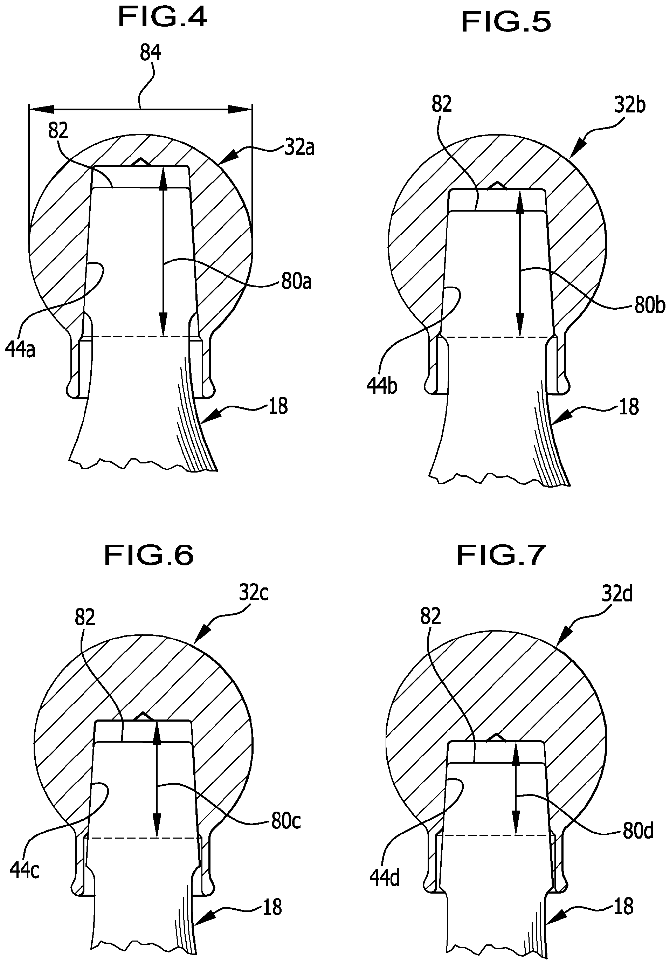

Das Hüftgelenkendoprothesensystem 10 umfasst bei einem Ausführungsbeispiel mehrere Kugelköpfe 32. In den Figuren 4 bis 7 sind vier unterschiedliche Kugelköpfe 32a, 32b, 32c und 32d beispielhaft dargestellt.

Jeder der Kugelköpfe 32a, 32b 32c und 32d weist einen Innenkonus 44a, 44b, 44c beziehungsweise 44d auf. Die Innenkonen 44a, 44b, 44c und 44d sind unterschiedlich tief ausgebildet.

Eine Tiefe 80a, 80b, 80c beziehungsweise 80d der Innenkonen 44a, 44b, 44c und 44d nimmt ausgehend vom Kugelkopf 32a bis zum Kugelkopf 32d sukzessive ab. Auf diese Weise kann eine Position des Gelenkzentrums 40 relativ zum Femurschaft 18 variiert werden. Je weiter ein freies Ende 82 in den jeweiligen Kugelkopf 32a, 32b, 32c beziehungsweise 32d hineinragt, umso weiter weg liegt das Gelenkzentrum 40 vom Ende 82.

Ein Außendurchmesser 84 der Kugelköpfe 32a, 32b, 32c und 32d ist bei den in den Figuren 4 bis 7 dargestellten Ausführungsbeispielen identisch. Dies ermöglicht es, alle Kugelköpfe 32, 32a, 32b, 32c und 32d mit derselben Gelenckopfschale 34 kugelgelenkig zu koppeln.

Die Gelenkkopfschale 34 definiert eine insbesondere von der Gelenkkopfschalenanschlagfläche 52 begrenzte Einfuhröffnung. Deren freier Querschnitt ist etwas kleiner als ein zum Außendurchmesser 84 korrespondierender Querschnitt der Kugelköpfe 32, 32a, 32b 32c und 32d. Dies ermöglicht es, die aus einem Kunststoff ausgebildete Gelenkkopfschale 34 auf einen der Kugelköpfe 32, 32a, 32b 32c beziehungsweise 32d aufzuschnappen.

Die Konusverbindung zwischen dem Kugelkopf 32 und dem Femurkonus 50 definiert eine Konushaltekraft. Bei einem Ausführungsbeispiel, bei dem das Schutzelement 54 mit dem Femurkonus 50 oder dem Femurhals 48 in Kontakt steht, wird eine Schutzelementhaltekraft definiert. Bei einem Ausführungsbeispiel liegt ein Verhältnis der Schutzelementhaltekraft und der Konushaltekraft in einem Bereich von etwa 1:20 bis etwa 1:500. Bei einem Ausführungsbeispiel liegt dieses Verhältnis in einem Bereich von etwa 1:50 bis etwa 1:200. Bei einem Ausführungsbeispiel beträgt das Verhältnis etwa 1:100.

Zum Verformen des Schutzelements 54 durch Anschlagen der Gelenkkopfschale 34 wird eine Verformungskraft benötigt. Bei einem Ausführungsbeispiel liegt ein Verformungsverhältnis der Verformungskraft und der Haltekraft der Konusverbindung in einem Bereich von etwa 1:20 bis etwa 1:500. Bei einem Ausführungsbeispiel liegt das Verformungsverhältnis in einem Bereich von etwa 1:50 bis etwa 1:200. Bei einem weiteren Ausführungsbeispiel beträgt das Verformungsverhältnis etwa 1:100.

Die beschriebenen Ausführungsbeispiele von Hüftgelenkendoprothesensystemen 10 ermöglichen es insbesondere, einen in einem Patienten implantierten Femurschaft 18 zu nutzen, um diesen mit einem der beschriebenen Gelencköpfe 14 zu koppeln. Unabhängig davon, wie der Femurschaft 18, insbesondere dessen Femurhals 48, ausgebildet ist, also insbesondere unabhängig davon, ob dieser eine hohe Rauheit sowie Ecken und Kanten aufweist, wird durch das Schutzelement 54 ein direkter Kontakt zwischen der Gelenkkopfschale 34 und dem Femurschaft 18, insbesondere dessen Femurhals 48, verhindert. Durch die besondere Oberflächenbehandlung der Außenfläche 74 wird die Gefahr einer Beschädigung der Gelenkkopfschale 34, insbesondere im Bereich von deren Gelenkkopfschalenanschlagfläche 52, minimiert.

### Bezugszeichenliste

- 10: Hüftgelenkendoprothesensystem
- 12: Gelenkpfanne
- 14: Gelenkkopf
- 16: Kugelgelenk
- 18: Femurschaft
- 20: Gelenkpfannenschale
- 22: Einsatzausnehmung
- 24: Gelenkpfanneneinsatz
- 26: Konusfläche
- 28: Konusfläche
- 30: Gelenkpfannenfläche
- 32, 32a, 32b, 32c, 32d: Kugelkopf
- 34: Gelenkkopfschale
- 36: Außenfläche
- 38: Innenfläche
- 40: Gelenkzentrum
- 42: Kugelkopffläche
- 44, 44a, 44b, 44c, 44d: Innenkonus
- 46: Längsachse
- 48: Femurhals
- 50: Femurkonus
- 52: Gelenkkopfschalenanschlagfläche
- 54: Schutzelement
- 56: Ringraum
- 58: Innenkonusfläche
- 60: Schutzelementinnenfläche
- 62: Verlängerungsfläche
- 64: Abstand
- 66: Abstand
- 68: Übergangsbereich
- 70: Ende
- 72: Dicke
- 74: Außenfläche
- 76: erste Schnittlinie
- 78: zweite Schnittlinie
- 80a, 80b, 80c, 80d: Tiefe
- 82: Ende
- 84: Außendurchmesser

## Patentansprüche

1. Hüftgelenkendoprothesensystem (10) umfassend eine Gelenkpfanne (12) und einen zur Gelenkpfanne (12) korrespondierend ausgebildeten Gelenkkopf (14) zur Ausbildung eines Kugelgelenks (16), welcher Gelenckopf (14) mit einem Femurschaft (18) koppelbar ist, wobei die Gelenkpfanne (12) eine Gelenkpfannenschale (20) mit einer Einsatzausnehmung (22) aufweist, in die ein Gelenkpfanneneinsatz (24) mit einer hohlkugeligen Gelenkpfannenfläche (30) eingesetzt ist, wobei der Gelenkkopf (14) zweiteilig ausgebildet ist und mindestens einen mit dem Femurschaft (18) koppelbaren Kugelkopf (32, 32a, 32b, 32c, 32d) und eine Gelenkkopfschale (34) umfasst und wobei die Gelenkkopfschale (34) einerseits mit dem mindestens einen Kugelkopf (32, 32a, 32b, 32c, 32d) kugelgelenkig und andererseits mit dem Gelenkpfanneneinsatz (24) kugelgelenkig gekoppelt ist, **dadurch gekennzeichnet, dass** der mindestens eine Kugelkopf (32, 32a, 32b, 32c, 32d) ein Schutzelement (54) umfasst zum Begrenzen einer Bewegung der Gelenkkopfschale (34) in Richtung auf den Femurschaft (18).

2. Hüftgelenkendoprothesensystem nach Anspruch 1, **dadurch gekennzeichnet, dass** das Schutzelement (54) und der mindestens eine Kugelkopf (32, 32a, 32b, 32c, 32d) einstückig ausgebildet sind.

3. Hüftgelenkendoprothesensystem nach einem der voranstehenden Ansprüche, **dadurch gekennzeichnet, dass** der mindestens eine Kugelkopf (32, 32a, 32b, 32c, 32d) einen Innenkonus (44, 44a, 44b, 44c, 44d) aufweist zum Ausbilden einer, insbesondere selbsthemmenden, Konusverbindung mit einem korrespondierenden Femurkonus (50) des Femurschafts (18).

4. Hüftgelenkendoprothesensystem nach Anspruch 3, **dadurch gekennzeichnet, dass** das Schutzelement (54) den Femurschaft (18) hülsenförmig umgibt.

5. Hüftgelenkendoprothesensystem nach Anspruch 3 oder 4, **dadurch gekennzeichnet, dass** das Schutzelement (54) vom Femurschaft (18), insbesondere einem Femurhals (48) desselben, beabstandet angeordnet ist zur Ausbildung eines Ringraums (56) zwischen dem Schutzelement (54) und dem Femurschaft (18).

6. Hüftgelenkendoprothesensystem nach einem der Ansprüche 3 bis 5, **dadurch gekennzeichnet, dass** der Innenkonus (44, 44a, 44b, 44c, 44d) eine Innenkonusfläche (58) definiert und dass eine in Richtung auf den Femurschaft weisende Schutzelementinnenfläche (60) des Schutzelements (54) von einer die Innenkonusfläche (58) virtuell verlängernden Verlängerungsfläche beabstandet ist.

7. Hüftgelenkendoprothesensystem nach Anspruch 5 oder 6, **dadurch gekennzeichnet, dass** ein Abstand (64) des Schutzelements (54) vom Femurschaft (18) und/oder ein Abstand (66) der virtuellen Verlängerungsfläche (62) von der Innenkonusfläche (58) maximal 1 mm beträgt.

8. Hüftgelenkendoprothesensystem nach einem der Ansprüche 3 bis 7, **dadurch gekennzeichnet, dass** sich der Innenkonus (44, 44a, 44b, 44c, 44d) im Übergangsbereich zum Schutzelement (54) im Innendurchmesser erweitert, insbesondere einstufig.

9. Hüftgelenkendoprothesensystem nach einem der voranstehenden Ansprüche, **dadurch gekennzeichnet, dass**
a) ein freies, vom mindestens einen Kugelkopf (32, 32a, 32b, 32c, 32d) weg weisendes Ende (70) des Schutzelements (54) wulstförmig verdickt ausgebildet ist, insbesondere von einer Längsachse (46) des Innenkonus (44) weg weisend,
und/oder
b) eine vom Femurschaft (18) weg weisende Außenfläche (74) des Schutzelements (54) poliert ist
und/oder
c) das Schutzelement (54) eine Dicke (72) aufweist, die maximal 1 mm beträgt.

10. Hüftgelenkendoprothesensystem nach einem der voranstehenden Ansprüche, **dadurch gekennzeichnet, dass**
a) der mindestens eine Kugelkopf (32, 32a, 32b, 32c, 32d) aus einem metallischen Werkstoff ausgebildet ist
und/oder
b) die Gelenkkopfschale (34) aus einem Kunststoff ausgebildet ist, insbesondere aus Polyethylen, weiter insbesondere aus Polyethylen mit ultrahohem Molekulargewicht (UHMWPE).

11. Hüftgelenkendoprothesensystem nach einem der voranstehenden Ansprüche, **dadurch gekennzeichnet, dass** eine Außenkontur der Gelenckopfschale (34) derart geformt ist, dass sie, wenn sie mit dem Schutzelement (54) in Kontakt steht, das Schutzelement (54) mindestens linienförmig, insbesondere flächig, berührt,
wobei insbesondere die Außenkontur der Gelenkkopfschale (34) eine konische, in Richtung auf den Femurhals (48) weisende Gelenkkopfschalenanschlagfläche (52) aufweist und dass eine erste Schnittlinie (76) der Gelenkkopfschalenanschlagfläche (52) mit einer Schnittebene, in der ein Gelenkzentrum (40) der kugelgelenkigen Verbindung zwischen der Gelenkkopfschale (34) und dem mindestens einen Kugelkopf (32, 32a, 32b, 32c, 32d) liegt, parallel oder im Wesentlichen parallel zu einer zweiten Schnittlinie (78) des Schutzelements (54) mit der Schnittebene verläuft.

12. Hüftgelenkendoprothesensystem nach einem der Ansprüche 3 bis 11, **dadurch gekennzeichnet, dass** das Hüftgelenkendoprothesensystem (10) zwei oder mehr Kugelköpfe (32, 32a, 32b, 32c, 32d) umfasst und dass Innenkonen (44, 44a, 44b, 44c, 44d) der zwei oder mehr Kugelköpfe (32, 32a, 32b, 32c, 32d) unterschiedlich tief ausgebildet sind,
wobei insbesondere ein von den zwei oder mehr Kugelköpfen (32, 32a, 32b, 32c, 32d) definierter Außendurchmesser (84) identisch oder im Wesentlichen identisch ist.

13. Hüftgelenkendoprothesensystem nach einem der voranstehenden Ansprüche, **dadurch gekennzeichnet, dass** die kugelgelenkige Verbindung zwischen der Gelenkkopfschale (34) und dem mindestens einen Kugelkopf (32, 32a, 32b, 32c, 32d) ein erstes Rotationszentrum (40) definiert, dass die kugelgelenkige Verbindung zwischen der Gelenkkopfschale (34) und dem Gelenkpfanneneinsatz (24) ein zweites Rotationszentrum (40) definiert und dass das erste Rotationszentrum (40) und das zweite Rotationszentrum (40) identisch oder im Wesentlichen identisch sind.

14. Hüftgelenkendoprothesensystem nach einem der voranstehenden Ansprüche, **gekennzeichnet durch** einen Femurschaft (18) mit einem Femurhals (48), an welchem ein Femurkonus (50) ausgebildet ist, welcher Femurkonus (50) ein freies Ende des Femurhalses (48) bildet.

15. Hüftgelenkendoprothesensystem nach Anspruch 14, **dadurch gekennzeichnet, dass**
a) ein Verhältnis einer Schutzelementhaltekraft zwischen dem Schutzelement (54) und dem Femurkonus (50) und/oder dem Femurhals (48) einerseits und einer Konushaltekraft der Konusverbindung andererseits in einem Bereich von etwa 1:20 bis etwa 1:500 liegt, insbesondere in einem Bereich von etwa 1:50 bis etwa 1:200, weiter insbesondere etwa 1:100 beträgt,
und/oder
b) ein Verformungsverhältnis einer Verformungskraft zum Verformen des Schutzelements (54) durch Anschlagen der Gelenkkopfschale (34) einerseits und einer Haltekraft der Konusverbindung andererseits in einem Bereich von etwa 1:20 bis etwa 1:500 liegt, insbesondere in einem Bereich von etwa 1:50 bis etwa 1:200, weiter insbesondere etwa 1:100 beträgt.

## Claims

1. Hip joint endoprosthesis system (10) comprising a joint socket (12) and a joint head (14) configured corresponding to the joint socket (12) for forming a ball joint (16), said joint head (14) being coupleable to a femoral shaft (18), wherein the joint socket (12) has a joint socket shell (20) with an insertion recess (22) into which a joint socket insert (24) with a hollow-spherical joint socket surface (30) is inserted, wherein the joint head (14) is of two-part configuration and comprises at least one joint head (32, 32a, 32b, 32c, 32d), which is coupleable to the femoral shaft (18), and a joint head shell (34), and wherein the joint head shell (34) is coupled to the at least one ball head (32, 32a, 32b, 32c, 32d) in a ball-jointed manner on the one hand and is coupled to the joint socket insert (24) in a ball-jointed manner on the other hand, **characterized in that** the at least one ball head (32, 32a, 32b, 32c, 32d) comprises a protective element (54) for delimiting a movement of the joint head shell (34) in the direction toward the femoral shaft (18).

2. Hip joint endoprosthesis system in accordance with Claim 1, **characterized in that** the protective element (54) and the at least one ball head (32, 32a, 32b, 32c, 32d) are formed in one piece.

3. Hip joint endoprosthesis system in accordance with any one of the preceding Claims, **characterized in that** the at least one ball head (32, 32a, 32b, 32c, 32d) has an inner cone (44, 44a, 44b, 44c, 44d) for forming an, in particular self-locking, cone connection with a corresponding femoral cone (50) of the femoral shaft (18).

4. Hip joint endoprosthesis system in accordance with Claim 3, **characterized in that** the protective element (54) surrounds the femoral shaft (18) in the shape of sleeve.

5. Hip joint endoprosthesis system in accordance with Claim 3 or 4, **characterized in that** the protective element (54) is arranged at a distance from the femoral shaft (18), in particular a femoral neck (48) thereof, for forming an annular space (56) between the protective element (54) and the femoral shaft (18).

6. Hip joint endoprosthesis system in accordance with any one of Claims 3 to 5, **characterized in that** the inner cone (44, 44a, 44b, 44c, 44d) defines an inner cone surface (58) and **in that** a protective element inner surface (60) of the protective element (54) pointing in the direction toward the femoral shaft is spaced at a distance from an extension surface virtually extending the inner cone surface (58).

7. Hip joint endoprosthesis system in accordance with Claim 5 or 6, **characterized in that** a distance (64) of the protective element (54) from the femoral shaft (18) and/or a distance (66) of the virtual extension surface (62) from the inner cone surface (58) is at most 1 mm.

8. Hip joint endoprosthesis system in accordance with any one of Claims 3 to 7, **characterized in that** the inner cone (44, 44a, 44b, 44c, 44d) expands in inner diameter, in particular in one step, in the transition region to the protective element (54).

9. Hip joint endoprosthesis system in accordance with any one of the preceding Claims, **characterized in that**
a) a free end (70) of the protective element (54) pointing away from the at least one ball head (32, 32a, 32b, 32c, 32d) is configured thickened in the shape of bead, in particular pointing away from a longitudinal axis (46) of the inner cone (44),
and/or
b) an outer surface (74) of the protective element (54) pointing away from the femoral shaft (18) is polished,
and/or
c) the protective element (54) has a thickness (72) that is at most 1 mm.

10. Hip joint endoprosthesis system in accordance with any one of the preceding Claims, **characterized in that**
a) the at least one joint head (32, 32a, 32b, 32c, 32d) is made of a metallic material,
and/or
b) the joint head shell (34) is made of a plastic material, in particular of polyethylene, further in particular of ultra-high molecular weight polyethylene (UHMWPE).

11. Hip joint endoprosthesis system in accordance with any one of the preceding Claims, **characterized in that** an outer contour of the joint head shell (32) is shaped in such a way that when it is in contact with the protective element (54), it contacts the protective element (54) at least linearly, in particular in surface-to-surface contact.
wherein, in particular, the outer contour of the joint head shell (34) has a conical joint head shell abutment surface (52) pointing in the direction toward the femoral neck (48), and **in that** a first section line (76) of the joint head shell abutment surface (52) with a section plane, in which a joint center (40) of the ball-jointed connection between the joint head shell (34) and the at least one ball head (32, 32a, 32b, 32c, 32d) lies, extends in parallel or substantially in parallel to a second section line (78) of the protective element (54) with the section plane.

12. Hip joint endoprosthesis system in accordance with any one of Claims 3 to 11, **characterized in that** the hip joint endoprosthesis system (10) comprises two or more ball heads (32, 32a, 32b, 32c, 32d) and **in that** inner cones (44, 44a, 44b, 44c, 44d) of the two or more ball heads (32, 32a, 32b, 32c, 32d) are configured having different depths, wherein, in particular, an outer diameter defined by the two or more ball heads (32, 32a, 32b, 32c, 32d) is identical or substantially identical.

13. Hip joint endoprosthesis system in accordance with any one of the preceding Claims, **characterized in that** the ball-jointed connection between the joint head shell (34) and the at least one ball head (32, 32a, 32b, 32c, 32d) defines a first rotational center (40), **in that** the ball-jointed connection between the joint head shell (34) and the joint socket insert (24) defines a second rotational center (40), and **in that** the first rotational center (40) and the second rotational center (40) are identical or substantially identical.

14. Hip joint endoprosthesis system in accordance with any one of the preceding Claims, **characterized by** a femoral shaft (18) with a femoral neck (48) on which a femoral cone (50) is formed, said femoral cone (50) forming a free end of the femoral neck (48).

15. Hip joint endoprosthesis system in accordance with Claim 14, **characterized in that**
a) a ratio of a protective element holding force between the protective element (54) and the femoral cone (50) and/or the femoral neck (48) on the one hand and a cone holding force of the cone connection on the other hand is in a range of about 1:20 to about 1:500, in particular in a range of about 1:50 to about 1:200, further in particular about 1:100,
and/or
b) a deformation ratio of a deformation force for deforming the protective element (54) by striking the joint head shell (34) on the one hand and a holding force of the cone connection on the other hand is in a range of about 1:20 to about 1:500, in particular in a range of about 1:50 to about 1:200, further in particular about 1:100.

## Revendications

1. Système d'endoprothèse de la hanche (10) comprenant un cotyle (12) et un condyle (14), conçu de façon à correspondre au cotyle (12), pour la conception d'une articulation sphérique (16), lequel condyle (14) peut être couplé avec une tige fémorale (18), dans lequel le cotyle (12) présente une coque de cotyle (20) avec un évidement d'insert (22) dans lequel est inséré un insert de cotyle (24) avec une surface de cotyle (30) sphérique et creuse, dans lequel le condyle (14) est conçu en deux parties et comprend au moins une tête sphérique (32, 32a, 32b, 32c, 32d), qui peut être couplée avec la tige fémorale (18), et une coque de condyle (34) et dans lequel la coque de condyle (34) est couplée d'une part côté par articulation sphérique avec la au moins une tête sphérique (32, 32a, 32b, 32c, 32d) et d'autre part par articulation sphérique avec l'insert de cotyle (24), **caractérisé en ce que** la au moins une tête sphérique (32, 32a, 32b, 32c, 32d) comprend un élément de protection (54) pour limiter un mouvement de la coque de condyle (34) dans la direction de la tige fémorale (18).

2. Système d'endoprothèse de la hanche selon la revendication 1, **caractérisé en ce que** l'élément de protection (54) et la au moins une tête sphérique (32, 32a, 32b, 32c, 32d) sont conçus en une seule pièce.

3. Système d'endoprothèse de la hanche selon l'une des revendications précédentes, **caractérisé en ce que** la au moins une tête sphérique (32, 32a, 32b, 32c, 32d) présente un cône intérieur (44, 44a, 44b, 44c, 44d) pour la conception d'une liaison conique, en particulier autobloquante, avec un cône de fémur (50) correspondant de la tige fémorale (18).

4. Système d'endoprothèse de la hanche selon la revendication 3, **caractérisé en ce que** l'élément de protection (54) enrobe sous forme de douille la tige fémorale (18).

5. Système d'endoprothèse de la hanche selon la revendication 3 ou 4, **caractérisé en ce que** l'élément de protection (54) est agencé à distance de la tige fémorale (18), en particulier d'un col de fémur (48) de celle-ci, pour la conception d'un espace annulaire (56) entre l'élément de protection (54) et la tige fémorale (18).

6. Système d'endoprothèse de la hanche selon l'une des revendications 3 à 5, **caractérisé en ce que** le cône intérieur (44, 44a, 44b, 44c, 44d) définit une surface de cône intérieur (58) et **en ce qu'**une surface intérieure d'élément de protection (60), orientée en direction de la tige fémorale, de l'élément de protection (54) est à distance d'une surface de prolongement prolongeant virtuellement la surface de cône intérieur (58).

7. Système d'endoprothèse de la hanche selon la revendication 5 ou 6, **caractérisé en ce qu'**une distance (64) de l'élément de protection (54) par rapport à la tige fémorale (18) et/ou une distance (66) de la surface de prolongement virtuelle (62) par rapport à la surface de cône intérieur (58) est au maximum de 1 mm.

8. Système d'endoprothèse de la hanche selon l'une des revendications 3 à 7, **caractérisé en ce que** le cône intérieur (44, 44a, 44b, 44c, 44d) s'élargit en diamètre intérieur, en particulier en une étape, dans la zone de transition vers l'élément de protection (54).

9. Système d'endoprothèse de la hanche selon l'une des revendications précédentes, **caractérisé en ce que**
a) une extrémité (70) libre, orientée en s'éloignant de la au moins une tête sphérique (32, 32a, 32b, 32c, 32d), de l'élément de protection (54) est conçue de façon épaissie en forme de boudin, en particulier orientée en s'éloignant d'un axe longitudinal (46) du cône intérieur (44),
et/ou
b) une surface extérieure (74), orientée en s'éloignant de la tige fémorale (18), de l'élément de protection (54) est polie
et/ou
c) l'élément de protection (54) présente une épaisseur (72) qui est au maximum de 1 mm.

10. Système d'endoprothèse de la hanche selon l'une des revendications précédentes, **caractérisé en ce que**
a) la au moins une tête sphérique (32, 32a, 32b, 32c, 32d) est conçue dans un matériau métallique
et/ou
b) la coque de condyle (34) est conçue dans un matériau, en particulier dans un polyéthylène, davantage en particulier dans un polyéthylène à poids moléculaire ultra élevé (UHMWPE).

11. Système d'endoprothèse de la hanche selon l'une des revendications précédentes, **caractérisé en ce qu'**un contour extérieur de la coque de condyle (34) est façonné de sorte que, lorsqu'il est en contact avec l'élément de protection (54), il effleure l'élément de protection (54) au moins sous la forme d'une ligne, en particulier en surface,
dans lequel en particulier le contour extérieur de la coque de condyle (34) présente une surface de butée de coque de condyle (52) conique, orientée en direction du col de fémur (48), et **en ce qu'**une première intersection (76) de la surface de butée de coque de condyle (52) avec un plan de coupe dans lequel un centre d'articulation (40) de la liaison par articulation sphérique se situe entre la coque de condyle (34) et la au moins une tête sphérique (32, 32a, 32b, 32c, 32d), se déroule en parallèle ou sensiblement en parallèle d'une deuxième intersection (78) de l'élément de protection (54) avec le plan de coupe.

12. Système d'endoprothèse de la hanche selon l'une des revendications 3 à 11, **caractérisé en ce que** le système d'endoprothèse de la hanche (10) comprend deux ou plusieurs têtes sphériques (32, 32a, 32b, 32c, 32d) et **en ce que** des cônes intérieurs (44, 44a, 44b, 44c, 44d) des deux ou plusieurs têtes sphériques (32, 32a, 32b, 32c, 32d) sont conçus avec des profondeurs différentes,
dans lequel en particulier un diamètre extérieur (84) défini par les deux ou plusieurs têtes sphériques (32, 32a, 32b, 32c, 32d) est identique ou sensiblement identique.

13. Système d'endoprothèse de la hanche selon l'une des revendications précédentes, **caractérisé en ce que** la liaison par articulation sphérique définit un premier centre de rotation (40) entre la coque de condyle (34) et la au moins une tête sphérique (32, 32a, 32b, 32c, 32d), **en ce que** la liaison par articulation sphérique définit un deuxième centre de rotation (40) entre la coque de condyle (34) et l'insert de cotyle (24) et **en ce que** le premier centre de rotation (40) et le deuxième centre de rotation (40) sont identiques ou sensiblement identiques.

14. Système d'endoprothèse de la hanche selon l'une des revendications précédentes, **caractérisé par** une tige fémorale (18) avec un col de fémur (48) au niveau duquel est conçu un cône de fémur (50), lequel cône de fémur (50) forme une extrémité libre du col de fémur (48).

15. Système d'endoprothèse de la hanche selon la revendication 14, **caractérisé en ce que**
a) un rapport d'une force de retenue d'élément de protection entre l'élément de protection (54) et le cône de fémur (50) et/ou le col de fémur (48) d'une part à une force de retenue de cône de la liaison conique d'autre part se situe dans une plage allant d'environ 1/20 à environ 1/500, en particulier dans une plage allant d'environ 1/50 à environ 1/200, davantage en particulier est d'environ 1/100,
et/ou
b) un rapport de déformation d'une force de déformation pour déformer l'élément de protection (54) par frappe de la coque de condyle (34) d'une part à une force de retenue de la liaison conique d'autre part se situe dans une plage allant d'environ 1/20 à environ 1/500, en particulier dans une plage allant d'environ 1/50 à environ 1/200, davantage en particulier est d'environ 1/100.
